# EUROPÄISCHE PATENTANMELDUNG

(11) **EP 4 483 855 A1**
(43) Veröffentlichungstag der Anmeldung: **01.01.2025**
(21) Anmeldenummer: 24182859.9
(22) Anmeldetag: 18.06.2024
(51) Int. Cl.: A61F 13/49, A61F 13/514, B32B 5/02

(54) **DECKLAGE FÜR EINEN EINWEG-HYGIENEARTIKEL UND EINWEG-HYGIENEARTIKEL**

(30) Priorität: 27.06.2023 DE 102023116947
(71) Anmelder: Nitto Advanced Film Gronau GmbH, 48599 Gronau (DE); NITTO DENKO CORPORATION, Ibaraki-shi Osaka 567-8680 (JP)
(72) Erfinder: Trinkaus, Jan Michael, 53881 Euskirchen (DE)
(74) Vertreter: Andrejewski - Honke Patent- und Rechtsanwälte Partnerschaft mbB

(57) **Zusammenfassung**

Die Erfindung betrifft eine Decklage für einen Einweg-Hygieneartikel, insbesondere eine Windel, umfassend eine Coextrusionsverbundfolie mit einem ersten vollflächigen Polymermaterial (1) und einem zweiten, elastischen Polymermaterial (2) , welches lediglich bereichsweise in Form zumindest eines Streifens zur Ausbildung zumindest eines elastischen ersten Funktionsabschnittes (3) mit dem zweiten, elastischen Polymermaterial (2) und zumindest eines zweiten Funktionsabschnitts (4) ohne das zweite, elastische Polymermaterial (2) angeordnet ist. Erfindungsgemäß ist vorgesehen, dass die Coextrusionsverbundfolie an dem zumindest einen Streifen gegenüber dem zumindest einen zweiten Funktionsabschnitt (4) eine erhöhte Dicke aufweist. Gegenstand der Erfindung ist auch ein Einweg-Hygieneartikel.

## Beschreibung

Die Erfindung betrifft eine Decklage für einen Einweg-Hygieneartikel, insbesondere eine Windel, umfassend eine Coextrusionsverbundfolie mit einem ersten vollflächigen Polymermaterial und einem zweiten, elastischen Polymermaterial, welches lediglich bereichsweise in Form zumindest eines Streifens zur Ausbildung zumindest eines elastischen ersten Funktionsabschnittes mit dem zweiten, elastischen Polymermaterial und zumindest eines zweiten Funktionsabschnitts ohne das zweite, elastische Polymermaterial angeordnet ist,. Gegenstand der Erfindung ist auch ein Einweg-Hygieneartikel.

Da elastische Komponenten weit teurer sind als nicht-elastische, beschränkt man gewöhnlich den Einsatz von elastischen Materialien und elastischen Abschnitten auf für den Komfort des Endnutzers von Hygieneprodukten essenziellen Bereichen, wie beispielsweise einen seitlichen Bereich einer Windel oder einem mittleren Bereich am Bündchen.

Dabei werden typischerweise elastische Komponenten an nicht-elastische Komponenten angeklebt oder angeschweißt. Beispielsweise können elastische Laminate zur Ausbildung von Windelohren an die Außenlage einer Windel angesetzt werden. Um den Einsatz elastischer Materialien zu minimieren, ist es auch bekannt, lediglich elastische Fäden oder Streifen einer elastischen Folie in einem Laminat anzuordnen oder direkt an der Außenlage zu befestigen.

Gemäß dem Stand der Technik werden elastische Laminate mit einer elastischen Folie und zumindest einer Decklage aus Nonwoven vorproduziert, zugeschnitten an einem separat gebildeten Windelkörper mit einer Außenlage, einer Innenlage und einem zwischen Außenlage und Innenlage angeordneten saugfähigen Kern befestigt.

Die Verfahren zum Anbringen solcher elastischen Laminate sind aufwendig. Die entsprechenden Zuschnitte müssen aus einer Endlosbahn geschnitten und dann präzise an dem Windelkörper angeordnet und befestigt werden. Häufig geschieht dies bei einer kontinuierlichen Bewegung der Windelkörper bei Geschwindigkeiten von etwa 100 bis 600 m/min (Meter pro Minute), wobei dann die Zuschnitte des elastischen Laminates entsprechend beschleunigt und geführt werden müssen. Zusätzlich ist häufig auch das Anbringen von Hakenbändern als Teil eines Verschlusssystems vorgesehen, was zu einer weiteren Erhöhung der Komplexität führt.

Eine fehlerhafte oder unzureichende Befestigung des elastischen Laminates an dem Windelkörper kann dazu führen, dass das elastische Laminat bei der Benutzung von dem Windelkörper abreißt, wodurch die entsprechende Windel unmittelbar unbrauchbar wird. Falls ein Abreißen während der Benutzung der Windel erfolgt, fällt dann auch unmittelbar die gewünschte Schutzfunktion weg. Dabei ist auch zu beachten, dass durch Produktionsfehler oder Produktionsschwankungen ein Abreißen auch vorzeitig vor Erreichen einer erwarteten Belastbarkeit erfolgen kann.

Wie bereits zuvor erläutert, ist eine vergleichsweise effiziente Nutzung elastischer Materialien möglich, wenn lediglich elastische Fäden zur Ausbildung elastischer Bereiche eingesetzt werden. Auch bei diesen Ansätzen ergeben sich relativ aufwendige und fehleranfällige Verfahren, insbesondere weil eine Vielzahl von Fäden gleichzeitig verarbeitet werden müssen. Des Weiteren können einzelne Fäden oder Streifen auch unerwünschte Druckspuren bei einem Benutzer hinterlassen.

Grundsätzlich ist es bekannt, mittels Coextrusion Laminate mit elastischen und nicht-elastischen Bereichen zu bilden. Ein erstes nicht-elastisches Polymermaterial und ein zweites, elastisches Polymermaterial können dabei als Streifen nebeneinander angeordnet werden, wozu auf die Druckschriften DE 198 06 452 A1, WO 2009/046556 A1, EP 2 411 212 B1, EP 2 533 964 B1 verwiesen wird.

Gemäß der WO 2008/073666 A2 können unterschiedliche elastische und nicht-elastische Polymermaterialien vielfältig angeordnet werden, wozu spezielle Extrusionsdüsen vorgesehen sind. Eine in ihrer äußeren Struktur gleichmäßige Coextrusionsverbundfolie kann so in ihrem Innern zur Funktionsoptimierung aus bereichsweise unterschiedlichen Polymermaterialien gebildet sein.

Aus der WO 2007/0785181 A1 ist es bekannt, dass ein Polymermaterial bei einer Coextrusionsverbundfolie lediglich bereichsweise vorgesehen ist, wobei dann die Zwischenräume von einem weiteren Polymermaterial aufgefüllt sind, welches sich auch mit einer geringen Schichtdicke über das vorgenannte Polymermaterial erstreckt.

Die beschriebenen Coextrusionsverbundfolien erweitern das Einsatzspektrum und erlauben im gewissen Maße eine Modulierung der mechanischen und insbesondere elastischen Eigenschaften. Gerade an Übergängen zwischen elastischen und nicht-elastischen Polymermaterialien besteht jedoch häufig die Gefahr eines Zerreißens.

Die Maßnahmen zur Herstellung der bekannten Coextrusionsverbundfolien sind vergleichsweise aufwendig. Des Weiteren sind auch die mechanischen Eigenschaften in Bezug auf bevorzugte Anwendungen an Einweg-Hygieneprodukten verbesserungsbedürftig.

Der vorliegenden Erfindung liegt die Aufgabe zugrunde, eine Decklage für einen Einweg-Hygieneartikel anzugeben, welche besonders vielseitig einsetzbar ist und die Fertigung eines Einweg-Hygieneartikel vereinfachen kann.

Des Weiteren soll auch ein entsprechender Einweg-Hygieneartikel angegeben werden.

Gegenstand der Erfindung und Lösung der Aufgabe sind eine Decklage gemäß Patentanspruch 1 sowie ein Einweg-Hygieneartikel 10.

Die Erfindung betrifft somit eine Decklage für einen Einweg-Hygieneartikel, insbesondere eine Windel, umfassend eine Coextrusionsverbundfolie mit einem ersten vollflächigen Polymermaterial und einem zweiten, elastischen Polymermaterial, welches lediglich bereichsweise in Form zumindest eines Streifens zur Ausbildung zumindest eines elastischen ersten Funktionsabschnittes mit dem zweiten, elastischen Polymermaterial und zumindest eines zweiten Funktionsabschnitts ohne das zweite, elastische Polymermaterial angeordnet ist. Erfindungsgemäß ist vorgesehen, dass die Coextrusionsverbundfolie an dem zumindest einen Streifen gegenüber dem zumindest einen zweiten Funktionsabschnitt eine erhöhte Dicke aufweist. Zweckmäßigerweise beträgt der Dickenverhältnis zumindest 1,2:1.

Erfindungsgemäß ist der zumindest eine erste Funktionsabschnitt elastisch im Sinne der Erfindung. Der zumindest eine zweite Funktionsabschnitt kann sowohl elastisch als auch nicht-elastisch sein. Bevorzugt sind in Rahmen der Erfindung Ausführungen, bei denen der zumindest eine zweite Funktionsabschnitt nicht-elastisch oder zumindest weniger elastisch als der zumindest eine erste Funktionsabschnitt ist. Das für die gute Elastizität des zumindest einen ersten Funktionsabschnittes vorgesehene zweite, elastische Polymer ist nur in Teilbereichen vorgesehen, wodurch Kostenersparnisse erreicht werden können. Lediglich das erste dann nicht-elastische oder weniger elastische erste Polymermaterial ist vollflächig angeordnet. Durch die erfindungsgemäß unterschiedliche Dicke der Coextrusionsverbundfolie werden im Rahmen der Erfindung weitere, nachfolgend erläuterte Vorteile erreicht.

Gemäß einer Weiterbildung ist vorgesehen, dass das Verhältnis einer an dem ersten Funktionsabschnitt bestimmten maximalen Foliendicke zu einer an dem zweiten Funktionsabschnitt bestimmten minimalen Foliendicke zumindest 1,4:1 beträgt.

Erfindungsgemäß ist vorgesehen, dass die Coextrusionsverbundfolie ein sich veränderndes Dickenprofil aufweist. Während sich das erste Polymermaterial vollflächig erstreckt, ist das zweite, vorzugsweise elastische Polymermaterial lediglich abschnittsweise vorgesehen. Dort ergibt sich durch die zusätzliche Anordnung des zweiten, vorzugsweise elastischen Polymermaterials eine erhöhte Dicke, wobei außerhalb des damit gebildeten elastischen Abschnitts in Summe weniger Material vorgesehen ist, was sich auch unmittelbar auf die Dicke auswirkt. Der zumindest eine zweite Funktionsabschnitt wird also nicht derart mit dem ersten Polymermaterial aufgefüllt, dass sich in Summe eine im Wesentlichen einheitliche Dicke der Folie ergibt, wie dies aus unterschiedlichem Stand der Technik bekannt ist.

Je nach Ausgestaltung kann die Decklage dazu vorgesehen sein, einen Körper des Einweg-Hygieneartikels im Wesentlichen vollständig von gegenüberliegenden Querrändern und gegenüberliegenden Längsrändern oder auch nur bereichsweise abzudecken.

Bei der vorzugsweise als Rücklage vorgesehenen Decklage ist gemäß einer weiteren bevorzugten Ausgestaltung vorgesehen, dass das zweite Polymermaterial in Form von zumindest und besonders bevorzugt genau zwei zu den Längsrändern benachbarten und in Längsrichtung verlaufenden Streifen angeordnet ist. Dabei kann vorgesehen sein, dass in einem entlang der Längsrichtung mittleren Bereich der Decklage durch seitliche Beineinschnitte die Streifen zumindest teilweise entfernt sind.

Dabei kann auch vorgesehen sein, dass die Streifen in zumindest einem an dem mittleren Bereich in Längsrichtung anschließenden Taillenbereich in die Decklage integrierte elastische Ohren bilden. Im Rahmen der beschriebenen Ausführung kann grundsätzlich vorgesehen sein, dass der zumindest eine elastische erste Folienabschnitt vor einer erstmaligen Benutzung vollständig oder bereichsweise vorgedehnt, d. h. aktiviert, ist, um danach eine leichte Dehnung mit einer weitgehenden elastischen Rückstellung zu erreichen. Eine solche Vordehnung kann beispielsweises durch Rollen mit ineinander greifenden Ringen erreicht werden. Entsprechende Maßnahmen sind dem Fachmann bekannt.

Des Weiteren ist es auch möglich, dass die Decklage nur Teile, beispielweise in Längsrichtung gesehene Enden abdeckt, während dazwischen ein mittlerer Bereich von einem anderen Material gebildet ist.

Die Coextrusionsverbundfolie der Decklage kann zumindest bereichsweise mit einem Nonwoven laminiert sein, um eine weiche Oberfläche zu bilden. Das Nonwoven kann sich dabei wie vorzugsweise auch die Coextrusionsverbundfolie über die gesamte Fläche der Decklage erstrecken oder lediglich bereichsweise angeordnet sein. Dabei kann es auch eine Rolle spielen, welche Bereiche mit einem Benutzer in Kontakt kommen, von einem Benutzer wahrgenommen werden oder für den Gesamteindruck des Einweg-Hygieneartikels von Bedeutung sind.

Ein weiterer Aspekt der Erfindung betrifft einen Einweg-Hygieneartikel, insbesondere eine Windel mit der zuvor beschriebenen Decklage. Der Einweg-Hygieneartikel weist dann bevorzugt die Decklage als vorzugsweise wasserdichte Außenlage auf, wobei des Weiteren eine wasserdurchlässige Innenlage und ein zwischen der Außenlage und der Innenlage angeordnete absorbierender Kern vorgesehen sind. Es ist auch möglich, dass die Decklage nur einen Teil der Außenlage bildet.

Gemäß einer bevorzugten Ausgestaltung ist dann vorgesehen, dass bei den Einweg-Hygieneartikel in Form einer Windel die Decklage an dem zumindest einen elastischen Abschnitt der Coextrusionsverbundfolie in einem Taillenbereich ein integriertes elastisches Bündchen oder Windelohr bildet.

Bei einer Windel als Einweg-Hygieneartikel kann die Coextrusionsverbundfolie auf unterschiedliche Weise mit weiteren Materialen und insbesondere Schichten aus Nonwoven kombiniert werden, wobei nachfolgend hierzu verscheiden Varianten exemplarisch erläutert sind.

Im Rahmen der Erfindung kann vorgesehen sein, dass eine Laminierung mit Nonwoven nur an elastischen Abschnitten und insbesondere in einem Bereich von Windelohren oder elastischen Bündchen einseitig oder beidseitig erfolgt. Gerade an elastischen Abschnitten kann durch eine zumindest einseitige Auflage von Nonwoven eine weiche Oberfläche erzeugt werden, welche auch zu einem Schutz eines Benutzers beitragen kann, um beispielsweise Hautirritationen zu vermeiden.

Alternativ kommt auch zumindest einseitig eine vollflächige Abdeckung der Coextrusionsverbundfolie mit einem Nonwoven in Betracht. Die Verbindung zwischen der Coextrusionsverbundfolie und dem zumindest einseitig vorgesehenen Nonwoven kann dabei abschnittsweise oder vollflächig erfolgen.

Sowohl bei einer abschnittsweisen oder vollflächigen Abdeckung mit Nonwoven können elastische Bereiche durch einen Vordehung, beispielsweise durch ineinander greifende Ringe, einen Reckrahmen oder der gleichen aktiviert werden. Eine weitgehend vollständige oder auch nur bereichsweise Dehnung der Coextrusionsverbundfolie kann auch durch zwei gegenüber eine Transportrichtung schräggestellte Scheiben erreicht werden, wobei eine solche Dehnungseinrichtung die Coextrusionsverbundfolie in einer Position aufnimmt, in der die schräggestellte Scheiben einander angenähert sind und dann durch den weiteren Transport an den Scheiben eine Aufweitung erfolgt. Gegebenenfalls können die ersten Funktionsabschnitte und die zweiten Funktionsabschnitte so ausgelegt sein, dass eine entsprechende Krafteinwirkung in wesentlich die ersten Funktionsabschnitte dehnt, während die Zugkräften an den zweiten Funktionsabschnitten ohne eine wesentliche Dehnung aufgenommen werden können.

Als Nonwoven sind beispielsweise ein kardiertes thermobondiertes Nonwoven, ein Spunbond-Nonwoven, oder auch ein Spunlace-Nonwoven geeignet. Auch können unterschiedliche Lagen von Nonwoven miteinander kombiniert sein, wie beispielsweise bei einem SMS-Nonwoven (Spundbond-Meltblown-Spunbund).

Denkbar ist auch eine streifenweise Laminierung, wobei der elastische Bereich mittels Klebstoff oder Ultraschall laminiert werden kann. Der nichtelastische Bereich, der vorzugsweise flüssigkeitsundurchlässig bleiben soll, kann dann vorzugsweise mittels Klebstoff laminiert werden.

Grundsätzlich ist es auch denkbar, eine durchgehende Schicht aus Nonwoven ein- oder beidseitig vorzusehen, wobei dann bereichsweise unterschiedliche Arten der Verbindung beispielsweise mittels Klebstoff. Thermobonding oder Ultraschallschweißen vorgesehen sein können. Die Art der Verbindung kann dann auf die jeweils vorgesehen Funktion und die zu erwartenden Belastungen angepasst werden. Bei der Einsatz von Klebstoff versteht es sich, dass auch bereichsweise verschiedene Verbindungsmuster und Flächengewichte vorgesehen sein können.

Wenn an einer Seite der Coextrusionsverbundfolie Nonwoven von bereichsweise aufgebacht wir, kann die gegenüberliegende Seite ohne Nonwoven, mit einem vollflächigen Nonwoven oder auch bereichsweise mit Nonwoven ausgeführt sein. Bei einer ebenfalls bereichsweisen Anordnung können die Bereiche an beiden Seiten in Deckung zueinander oder zueinander versetzt sein.

Bereiche des insbesondere als Windel ausgestalteten Einweg-Hygieneartikels könnte zur Verbesserung des Luftaustausches perforiert werden, z.B. mittels Laser oder Vakuumperforation der Coextrusionsverbundfolie. Bei einer Laminierung mit zumindest einem Nonwoven kann eine solche Perforation vor oder nach der Laminierung erfolgen.

Bei einer bevorzugten Ausgestaltung werden bei einer Windel an beiden Seiten elastische Windelohren oder elastische Bündchenabschnitte vorgesehen, wobei dazu wie beschrieben zwei elastische Folienabschnitte der Coextrusionsverbundfolie vorgesehen sind. Grundsätzlich ist es alternativ aber auch denkbar, einen elastischen Folienabschnitt in die Mitte eines Windelkörpers zu legen. Gegebenenfalls sind dann jedoch Anpassungen im Bereich eines saugfähigen Kerns notwendig, damit dieser nicht bei einer zu starken Dehnung in seiner Funktion beeinträchtigt wird. Denkbar wäre es beispielsweise in Bereich des Kerns auf eine Aktivierung zu verzichten, ggf. durch eine weitere Materiallage eine ausreichende Festigkeit sicherzustellen und/oder die Coextrusionsverbundfolie bei der Auflage des Kern im Herstellungsprozess zu Dehnen (entsprechend eines "Stretch-bonding"), damit der Kern bei der Nutzung nicht auf Zug belastet wird.

Eine Kombination mit Decksichten aus Nonwoven oder dergleichen kann auch an dem zumindest einen gewünschten elastischen Abschnitt in einem gedehnten Zustand (entsprechend eines "Stretch-bonding") erfolgen. Bei lediglich einem ersten Funktionsabschnitt an der Windel befindet sich dann dieser Bereich an dem Bauch und/oder dem Rücken, eines Benutzers.

Zusätzlich oder alternativ zu einer Aktivierung oder einem Stretch-bonding kann die Coextrusionsverbundfolie auch mit Materialien kombiniert werden, welche selbst elastisch sind oder besonders leicht auch ohne eine Aktivierung dehnbar sind. In Betracht kommen beispielsweise eine elastischer Kern und/oder ein elastisches Nonwoven als Decklage.

Gemäß einer bevorzugten Weiterbildung hinsichtlich der Coextrusionsverbundfolie ist vorgesehen, dass das Verhältnis einer an dem zweiten Funktionsabschnitt bestimmten maximalen Gesamtdicke des ersten Polymermaterials zu einer an dem ersten Funktionsabschnitt bestimmten minimalen Gesamtdicke des ersten Polymermaterials zumindest 2:1 beträgt

Dieser Aspekt bezieht sich auf die Verteilung des ersten Polymermaterials, welches sich zwar vollständig erstreckt, aber bereichsweise mit einer unterschiedlichen Dicke vorgesehen ist. Wenn gemäß einer bevorzugten Ausgestaltung der Erfindung das erste Polymermaterial das beispielsweise als Streifen vorgesehene zweite, elastische Polymermaterial beidseitig abdeckt, bezieht sich die Gesamtdicke dann auf die Summe der Dicke des an beiden Seiten des zweiten, vorzugsweise elastischen Polymermaterials vorhandenen erstem Polymermaterial.

Gerade bei einer elastischen Ausgestaltung des zweiten Polymermaterials ergeben sich wie nachfolgend weiter beschrieben besondere Vorteile. Insbesondere kann dann vorgesehen sein, dass zweiten Polymermaterial elastisch ist, wobei der erste Funktionsabschnitt einen elastischen Folienabschnitt und der zweite Funktionsabschnitt einen nicht-elastischen Folienabschnitt bilden.

In Kombination mit den zuvor beschriebenen Dickenverhältnissen ist dann vorgesehen, dass das erste Polymermaterial an dem zumindest einen elastischen Folienabschnitt mit einer geringeren Gesamtdicke als an dem nicht-elastischen Folienabschnitt vorgesehen ist. Wie nachfolgend weiter erläutert ist das erste Polymermaterial im Rahmen der Erfindung vorzugsweise weniger elastisch als das zweite Polymermaterial, wobei das erste Polymermaterial bzw. damit gebildeten Schichten insgesamt im Rahmen der Erfindung bevorzugt nicht-elastisch sind.

Das Dickenverhältnis des ersten Polymermaterials ist gemäß der beschriebenen Ausführungsform also gegenläufig zu der variierenden Foliendicke. Bezogen auf ein durchschnittliches Flächengewicht oder eine durchschnittliche Gesamtdicke des ersten Polymermaterials ist dieses an dem zumindest einen elastischen Folienabschnitt ausgedünnt.

Dabei ist grundsätzlich zu beachten, dass dann das erste nicht-elastische oder weniger elastische Polymermaterial zu erhöhten Zugkräften an dem zumindest einen elastischen Folienabschnitt und auch einer höheren bleibenden Verformung führen kann, was in der Regel unerwünscht ist. Eine dünne Schicht des ersten Polymermaterials auf dem zweiten, gemäß der beschriebenen bevorzugten Ausgestaltung elastischen Polymermaterial ist zwar hinsichtlich der Verbundfestigkeit und häufig auch für die Anordnung weiterer Lagen wie beispielsweise Schichten aus Nonwoven von Vorteil, diese Funktionalität wird aber bereits bei einer geringen Schichtdicke erreicht. Dadurch dass das erste Polymermaterial an dem zumindest einen elastischen Folienabschnitt nur in einer geringen Menge vorhanden ist, werden die beschriebenen negativen Einflüsse reduziert.

Auch wenn die Coextrusionsverbundfolie an dem nicht-elastischen Folienabschnitt eine geringere Foliendicke als an dem zumindest einen elastischen Folienabschnitt aufweist, liegt dort das erste Polymermaterial mit einer größeren Gesamtdicke als an dem zumindest einen elastischen Folienabschnitt vor. Dort, wo das zweite, elastische Polymermaterial nicht vorhanden ist, kann es nicht zur mechanischen Stabilität und Festigkeit der Coextrusionsverbundfolie beitragen, weshalb dort die erhöhte Schichtdicke des ersten Polymermaterial von Vorteil ist. Unter Berücksichtigung der üblichen Eigenschaften von elastischen und nicht-elastischen Polymeren sowie der Anforderungen an die Coextrusionsverbundfolie ist die Foliendicke dennoch wesentlich geringer als die an dem zumindest eine elastischen Folienabschnitt bestimmte maximale Foliendicke, wodurch sich auch eine besonders gute und effiziente Materialausnutzung und Kosteneffektivität ergibt. Gerade bei Einweg-Hygieneartikeln sind ginge Kosten und eine gute Materialausnutzung aus wirtschaftlicher und ökologische Sicht von besonderer Bedeutung.

Die Coextrusionsverbundfolie ist gemäß einer bevorzugten Ausgestaltung der Erfindung vollständig oder im Wesentlichen aus dem ersten Polymermaterial und dem zweiten Polymermaterial gebildet, wobei gegebenenfalls jedoch auch dünne, in Coextrusionsprozess eingebrachte Haftvermittlerschichten oder dergleichen vorgesehen sein können.

Gemäß einer alternativen Ausgestaltung umfasst die Coextrusionsverbundfolie ein drittes Polymermaterial, welches separat zu dem ersten Polymermaterial und dem zweiten Polymermaterial als zumindest eine Schicht in der Coextrusionsverbundfolie vorgesehen ist.

Bevorzugt ist das dritte Polymermaterial an dem zumindest einen ersten Funktionsabschnitt zwischen dem ersten Polymermaterial und dem zweiten Polymermaterial angeordnet ist.

Das dritte Polymermaterial kann genutzt werden, um bei der Coextrusionsverbundfolie weitere Funktion und/oder weitere Funktionsabschnitte zu ermöglichen.

Bevorzugt ist das dritte Polymermaterial bezogen auf eine Produktionsrichtung und eine dazu senkrechte Querrichtung der Coextrusionsverbundfolie überall dort vorgesehen, wo auch das zweite, vorzugsweise elastische Polymermaterial vorgesehen ist.

Davon ausgehend kann sich das dritte Polymermaterial wie das erste Polymermaterial vollflächig erstrecken. Denkbar ist beispielsweise, dass mit dem zweiten Polymermaterial der zumindest eine erste Funktionsabschnitt mit sehr ausgeprägten elastischen Eigenschaften gebildet wird. Dann ist es möglich, dass das dritte Polymermaterial eine geringere Elastizität als das zweite Polymermaterial aufweist, um auch außerhalb des zumindest einen ersten Funktionsabschnitts gewisse elastische Rückstellkräfte bereitstellen zu können. Bei der vollflächigen Erstreckung des ersten und dritten Polymermaterials bilden diese dann den zumindest einen zweiten Funktionsabschnitt, welcher je nach Ausgestaltung zumindest weniger elastisch als der zumindest eine erste Funktionsabschnitt ist. Das erste Polymermaterial kann dann dort als nicht-elastische Außenschicht vorgesehen sein.

Gemäß einer bevorzugten Ausgestaltung der Erfindung ist vorgesehen, dass der zumindest eine erste Funktionsabschnitt in Form eines Streifens in der Produktionsrichtung verläuft. Ein solcher Streifen kann bei einer Coextrusion dadurch erreicht werden, dass eine Schmelze des zweiten Polymermaterials lediglich bereichsweise vorgesehen ist, wobei ein bevorzugtes Verfahren zur Herstellung der Coextrusionsverbundfolie nachfolgend erläutert wird.

Im Rahmen der Erfindung ist es des Weiteren bevorzugt, wenn mehrere vorzugsweise elastische erste Funktionsabschnitte vorgesehen sind, welche beispielsweise jeweils einen Streifen bilden.

Zumindest ein Streifen oder vorzugsweise zumindest zwei Streifen können senkrecht zu der Produktionsrichtung beispielsweise eine Breite zwischen 1 cm und 12 cm aufweisen. Die Breite der Streifen ergibt sich dabei aus der Breite, über die sich das zweite, vorzugsweise elastische Polymermaterial senkrecht zur Produktionsrichtung erstreckt.

Dabei ist zu beachten, dass sich auch über die Breite eines einzelnen Streifens die Gesamtdicke des ersten Polymermaterials, die Gesamtdicke des zweiten Polymermaterials, ggf. die Gesamtdicke des dritten Polymermaterials sowie die Foliendicke in einem gewissen Maße ändern können.

Üblicherweise ergibt sich für die genannten Werte ein gewisses Profil, welches sich aus dem Fließverhalten der Polymermaterialien und insbesondere deren Viskosität bei dem Herstellungsprozess ergibt. Wie auch nachfolgend weiter erläutert, sind in diesem Zusammenhang auch die Schmelze-Massefließraten (MFR) von Bedeutung.

Gemäß einer Weiterbildung der Erfindung ist vorgesehen, dass zumindest zwei vorzugsweise elastische erste Funktionssabschnitte in Form von Streifen in der Produktionsrichtung verlaufen, wobei der Abstand zwischen den Streifen zwischen 3 cm und 24 cm beträgt. Insbesondere kann eine Vielzahl von Streifen mit einem dann gleichmäßigen Abstand oder im Wesentlichen gleichmäßigen Abstand zwischen den Streifen vorgesehen sein, um beispielsweise mehrnutzig Materialzuschnitte für Einweg-Hygieneprodukte zu bilden.

Die angegebene bevorzugte Breite der Streifen sowie des bevorzugten Abstandes zwischen den Streifen erlaubt eine unterschiedliche Skalierung. Beispielsweise ist eine solche unterschiedliche Skalierung zweckmäßig, um Windeln für Babys in unterschiedlichen Altersklassen oder, beispielsweise Inkontinenzartikel für Erwachsene mit unterschiedlichen Körpermaßen bereitstellen zu können. Dabei kann es besonders zweckmäßig sein, eine Breite im unteren bzw. oberen Bereich der angegebenen Spanne mit einem Abstand ebenfalls in einem unteren bzw. oberen Bereich der dafür angegebenen Spanne zu kombinieren. Das Verhältnis der Breite der Streifen zu dem Abstand der Streifen und damit das Verhältnis der elastischen Abschnitte zu den nicht-elastischen Abschnitten kann beispielsweise bei 1:1 bis 1:3 liegen.

Im Rahmen der Erfindung weist die Coextrusionsverbundfolie vorzugsweise zumindest einen elastischen Folienabschnitt und einen nicht-elastischen Folienabschnitt auf. Im Rahmen der Erfindung wird ein Folienabschnitt als elastisch bezeichnet, wenn bei einer Dehnung von 100 % bezogen auf eine Ausgangslänge eine Rückstellung um zumindest 50 % bezogen auf die Ausgangslänge erfolgt. Vorzugsweise weist der elastische Abschnitt bei einer Dehnung um 100 % eine elastische Rückstellung um zumindest 70 % auf.

Ein Folienabschnitt wird im Rahmen der Erfindung als nicht-elastisch bezeichnet, wenn bei einer Dehnung um 100 % bezogen auf eine Ausgangslänge eine Rückstellung von weniger als 50 % bezogen auf die Ausgangslänge erfolgt. Vorzugsweise ist nach einer Dehnung von 100 % die Rückstellung weniger als 30 %.

Gemäß einer bevorzugten Ausgestaltung der Erfindung ist vorgesehen, dass an dem zumindest einen ersten Funktionsabschnitt das zweite, vorzugsweise elastische Polymermaterial beidseitig von dem ersten Polymermaterial abgedeckt ist. An dem zumindest einen ersten Funktionsabschnitt ergibt sich dann ein zumindest dreischichtiger Aufbau, wobei insbesondere aber auch dünne Haftvermittlerschicht vorgesehen sein können. Das erste Polymermaterial kann dazu bei der Herstellung beidseitig als Polymerschmelze zugeführt werden.

Bei der beschriebenen Ausgestaltung ergibt sich dann an dem zumindest einen nicht-elastischen Abschnitt eine im Wesentlichen einheitliche Struktur, die von dem ersten Polymermaterial gebildet ist. Gegebenenfalls ist jedoch bei einer Analyse noch feststellbar, wenn das erste Polymermaterial beidseitig zugeführt wird. Dies gilt insbesondere, wenn zur Erhöhung der Verbundhaftung Haftvermittlerschichten vorgesehen sind, welche dann auch an dem nicht-elastischen Abschnitt innenliegende Schichten bzw. eine gemeinsame innenliegende Schicht bilden.

Wie zuvor erläutert, kann auch ein drittes Polymermaterial vorgesehen sein. Dann ist vorzugsweise an dem zumindest einen ersten Funktionsabschnitt vorzugsweise ein fünfschichtiger Aufbau mit dem zweiten Polymermaterial in der Mitte, dem daran beidseitig angrenzenden dritten Polymermaterial und schließlich dem ersten Polymermaterial aus außenliegende Schichten vorgesehen.

Im Rahmen der Erfindung kann insbesondere vorgesehen sein, dass die Coextrusionsverbundfolie einen bezüglich einer Mittellinie symmetrischen oder im Wesentlichen symmetrischen Schichtaufbau aufweist.

Wie auch zuvor erläutert, kann der Coextrusionsfolienverbund an dem zumindest einen zweiten, vorzugsweise nicht-elastischen Funktionsabschnitt vollständig oder im Wesentlichen aus dem ersten und/oder dem dritten Polymermaterial gebildet sein. Bevorzugt ist grundsätzlich, dass der zumindest eine vorzugsweise nicht-elastische zweite Funktionsabschnitt zu zumindest 70 Gew.-% aus dem ersten und/oder dritten Polymermaterial gebildet ist. Weitere funktionelle Schichten zum Zwecke der Versteifung oder dergleichen sind bevorzugt nicht vorgesehen. Wenn im Rahmen der beschriebenen Varianten der zweite Funktionsabschnitt von dem ersten und dem dritten Polymermaterial gebildet ist, kann das erste Polymermaterial beidseitig des dritten Polymermaterials in Form von dünnen Außenschichten vorgesehen sein.

Das erste Polymermaterial, das zweite Polymermaterial und ggf. das dritte Polymermaterial können jeweils aus einem einzigen Polymertyp mit einer entsprechenden Produktbezeichnung gebildet sein. Grundsätzlich kann für das erste Polymermaterial und/oder das zweite Polymermaterial und/oder das ggf. vorgesehene Polymermaterial ein Blend vorgesehen sein, wobei auch verschiedene Zusatz- und Füllstoffe zugesetzt sein können. Der Begriff Polymermaterial bezieht sich dabei also auf das aus der Schmelze extrudierte Polymers bzw. Polymergemisch einschließlich etwaiger Zusatzstoffe.

Das erste Polymermaterial weist gemäß einer bevorzugten Ausgestaltung der Erfindung ein Polyolefin oder mehrere Polyolefine als Polymeren Hauptbestandteil auf. Neben Polymeren Komponenten kommen auch Füllstoffe in Betracht, die je nach Anwendungsfall mit unterschiedlichen Gewichtsanteilen vorgesehen sein können. Füllstoffe können je nach Anforderungen zur Verbesserung der Eigenschaften und/oder zur Reduzierung der Kosten beitragen. Insbesondere für den Einsatz beim Einweg-Hygieneartikel, beispielsweise als Rücklage einer Windel, kann Talkum vorgesehen sein. Talkum als pulverisierte Form des Minerals Talk gehört zu der Gruppe der Schichtsilikate, wobei grundsätzlich auch andere Schichtsilikate im Rahmen der Erfindung als Füllstoffe in Betracht kommen. Talkum ist kostengünstig und kann als Schichtsilikat zu einer Verbesserung der mechanischen Eigenschaften beitragen. Des Weiteren kann Talkum dazu genutzt werden, um nach einem Recken eine erhöhte Atmungsaktivität zu erreichen, wobei sich an den Partikeln des Schichtsilikats gewisse Poren und Öffnungen ergeben können. Um eine besonders hohe Aktivität zu erreichen, sind auch Blends mit einem hohen Kreideanteil vorgesehen, wobei dann durch ein Recken besonders viele Poren erzeugt werden können

Sofern Schichtsilikate oder andere Füllstoffe vorgesehen sind, so können diese beispielsweise mit einem Anteil von 4 Gew.-% bis 70 Gew.-%, insbesondere 5 Gew.-% bis 20 Gew.-% vorhanden sein. Ein besonders hoher Anteil von mehr als 40 Gew.-% ist insbesondere bei Kreide als Füllstoff vorgesehen.

Neben derartigen Füllstoffen können auch weitere Zusätze wie Farbstoffe, UV-Stabilisatoren, Bearbeitungsmittel oder dergleichen vorgesehen sein. Entsprechende organische und/oder vorzugsweise anorganische funktionale Zusatzstoffe sind vorzugsweise mit einem Anteil von weniger als 10 Gew.-%, insbesondere weniger als 5 Gew.-% vorgesehen.

Als polymerer Bestandteil des ersten Polymermaterials sind insbesondere Polyethylene und Polypropylene geeignet.

Wie bereits zuvor dargelegt, werden insbesondere mit dem ersten Polymermaterial die zweiten Funktionsabschnitte bei einem Teil der beschriebenen Ausgestaltungen vollständig oder zu einem großen Anteil gebildet. Obwohl die zweiten Funktionsabschnitte wie zuvor definiert vorzugsweise keine ausgeprägten elastischen Eigenschaften aufweisen, kann eine gewisse Beimischung von, beispielsweise 3 Gew.-% bis 30 Gew.-%, insbesondere 5 Gew.-% bis 20 Gew.-% thermoplastischem Polyolefin-Elastomer (TPO) bei der ersten Polymerkomponente zweckmäßig sein. Diese Anteile sind nicht dazu vorgesehen und auch nicht ausreichend, um den entsprechenden Bereichen im Sinne der Erfindung elastische Eigenschaften zu verleihen. Orientierende Versuche zeigen jedoch, dass an den Übergängen zwischen dem zumindest einen vorzugsweise elastischen ersten Funktionsabschnitt und dem zumindest einen vorzugsweise nicht-elastischen zweiten Funktionsabschnitt die Gefahr eines Zerreißens bei einer Zugbelastung teilweise signifikant reduziert werden kann.

Das zweite, vorzugsweise elastische Polymermaterial kann zumindest einen Styrol-Block-Copolymer und/oder zumindest ein Polyolefin-Elastomer enthalten. Zu den geeigneten Styrol-Block-Copolymeren zählen beispielsweise StyrolButadien-Styrol (SBS), Styrol-Isopren-Styrol (SIS), Styrol-Ethen-Butien (SEBS) und Styrol-Ethen-Propen-Styrol (SEPS). Gemäß einer bevorzugten Ausgestaltung weist das erste Polymermaterial 60 bis 90 Gew.-% SBS auf. Geeignet sind beispielsweise SBS-Typen mit einem Styrol/Butadien Verhältnis in einem Bereich von 20:80 bis 40:60, beispielsweise 30:70.

Sofern das dritte Polymermaterial vorgesehen ist, ist dafür ein Polyolefin-Elastomer (TPE-O) bevorzugt. Bevorzugt erfolgt dann eine Kombination mit dem zuvor beschriebenen ersten nicht-elastischen Polymermaterial auf der Basis von nicht-elastischem Polyolefin und dem zweiten Polymermaterial auf der Basis von Styrol-Block-Copolymer.

Erfindungsgemäß ist das Verhältnis der an dem zumindest einen ersten Funktionsabschnitt bestimmten maximalen Foliendicke zu der an dem zumindest einen zweiten Funktionsabschnitt bestimmten minimalen Folie Dicke zumindest 1,2:1. Das Verhältnis liegt üblicherweise in einem Bereich zwischen 1,4:1 und 3:1, insbesondere in einem Bereich zwischen 1,5:1 und 2:1.

Die maximale Gesamtdicke kann beispielsweise zwischen 25 µm und 75 µm, insbesondere zwischen 30 µm und 50 µm betragen.

Die minimale Gesamtdicke kann beispielsweise zwischen 10 µm und 40 µm, insbesondere zwischen 19 µm und 31 µm betragen.

Vorzugsweise ist vorgesehen, dass das Verhältnis der an dem zweiten Funktionsabschnitt bestimmten maximalen Gesamtdicke des ersten Polymermaterials zu dem an dem ersten Funktionsabschnitt bestimmten minimalen Dicke des ersten Polymermaterial zumindest 2:1 beträgt. Besonders bevorzugt beträgt das Verhältnis zumindest 3:1. Üblicherweise liegt das Verhältnis in einem Bereich zwischen 2:1 und 9:1.

Auch hinsichtlich eines möglichen erfindungsgemäße Herstellungsverfahrens sind die Schmelze- Massefließraten der ersten Polymerkomponente und der zweiten, elastischen Polymerkomponente und insbesondere auch deren Verhältnis relevant.

Gemäß einer bevorzugten Ausgestaltung der Erfindung ist vorgesehen, dass das Verhältnis der Schmelze- Massefließraten (MFR) bei 200 °C und 5 kg des ersten Polymermaterials und des zweiten Polymermaterial bei 1:1 bis 2:1 liegt. Die Schmelze-Massefließraten beziehen sich dabei auf das erste und das zweite Polymermaterial, also - wie zuvor erläutert - auf das aus der Schmelze extrudierte Polymer bzw. Polymergemisch einschließlich etwaiger Zusatzstoffe.

Die Definition der Schmelze- Massefließraten bezieht sich dabei auf die Norm ASTM D1238 in der zum Anmeldetag gültigen Fassung unter Berücksichtigung der konkret angegebenen Prüftemperatur von 200° C und Prüflast von 5 kg.

Für das zweite Polymermaterial kann die Schmelze-Massefließraten beispielsweise in einem Bereich von 14 bis 27 g/10 min (200 °C/5 kg) liegen, wobei sich dann die Schmelze- Massefließraten des ersten Polymermaterial anhand des zuvor angegebenen Verhältnisses ergibt.

Sofern das dritte Polymermaterial vorgesehen ist, liegt das Verhältnis der Schmelze-Massefließraten (MFR) bei 200 °C und 5 kg des dritten Polymermaterials und des zweiten Polymermaterial vorzugsweise bei 2:1 bis 1:2, insbesondere 1:1 bis 2:1.

Die Coextrusionsverbundfolie kann grundsätzlich auf unterschiedliche Weise gebildet werden. Erfindungswesentlich ist dabei, dass die beschriebenen Verhältnisse der Foliendicke sowie des ersten Polymermaterial in Bezug auf den elastischen Abschnitt und den nicht-elastischen Abschnitt erreicht werden.

Grundsätzlich ist es denkbar, in einer Extrusionsdüse an dem Austrittsspalt eine spezielle Konturierung vorzusehen und/oder innerhalb der Coextrusiondüse die Polymermaterialien in geeigneter Weise zuzuführen.

Besonders bevorzugt ist ein erfindungsgemäßes Verfahren zur Herstellung einer Coextrusionsverbundfolie gemäß Patentanspruch 17.

Dabei werden in einer Extrusionsdüse eine Schmelze des ersten Polymermaterials vollflächig und eine Schmelze des zweiten Polymermaterials lediglich bereichsweise in Form von zumindest einem Streifen geführt, wobei das erste Polymermaterial und das zweite Polymermaterial derart gemeinsam mit der Extrusionsdüse entlang einer Produktionsrichtung extrudiert werden, dass die Coextrusionsverbundfolie mit einem entlang einer Querrichtung variierenden Dickenprofil gebildet wird.

Die Coextrusionsverbundfolie wird direkt in einem einheitlichen Coextrusionsprozess gebildet. Um dabei die beschriebenen Dickenverhältnisse zu erlangen, können verschieden Maßnahmen und Eigenschaften genutzt und auch miteinander kombiniert werden. Wie auch nachfolgend weiter erläutert, kann ein Düsenspalt insbesondere eine Cast-Düse an seinem Ausgang dauerhaft so vorgeformt sein, dass durch eine Profilierung des Spaltes mit Engstellen und Erweiterungen die gewünschte Profilierung der Coextrusionsverbundfolie selbst zumindest teilweise vorgegeben wird. Die Profilierung ist dabei auf die Zuführung des ersten Polymermaterials und des lediglich abschnittsweise vorgesehenen zweiten Polymermaterials abzustimmen und dann weitgehend festgelegt.

Zusätzlich oder alternativ kann die Coextrusion auch mit einer Cast-Düse durchgeführt werden, welche eine verstellbare Düsenlippe aufweist. Während üblicherweise die Düsenlippe in Stand der Technik dazu genutzt wird, über eine Folienbreite eine gleichmäßige Foliendicke zu erreichen, kann die Düsenlippe im Rahmen der Erfindung gezielt dazu genutzt werden, um die erfindungsgemäße Profilierung der Coextrusionsverbundfolie zumindest teilweise vorzugeben bzw. zu unterstützen. Auch dabei ist dann die Einstellung der Düsenlippe auf die Zuführung des ersten Polymermaterials und des lediglich abschnittsweise vorgesehenen zweiten Polymermaterials abzustimmen. Bei einer Änderung beispielsweise der Breite und/oder des Abstandes von Streifen des zweiten Polymermaterials kann dann die Düsenlippe in geeigneter Weise verstellt werden.

Bei der Coextrusion ergibt sich bei dem Verlassen der Extrusionsspaltes durch den damit verbundenen Druckabfall eine Aufweitung der Schmelze, welche auch als Strangaufweitung bezeichnet wird. In diesem Zusammenhang kann auch eine unterschiedliche Schmelzeelastizität der ersten Polymerkomponente und der zweiten Polymerkomponente genutzt werden, um zumindest teilweise die gewünschte Profilierung der Coextrusionsverbundfolie. Auch bei den exemplarisch zuvor genannten Maßnahmen ist die Schmelzeelastizität zweckmäßigerweise zumindest zu berücksichtigen.

Gemäß einer konkreten Ausgestaltung können für einen dreischichtigen Aufbau an dem zumindest einen ersten Funktionsabschnitt an der Cast-Düse drei übereinander angeordnete Kleiderbügelverteiler vorgesehen sein, wobei das zweite Polymermaterial dem mittleren Kleiderbügelverteiler zugeführt wird. Der mittlere Kleiderbügelverteiler weist Stauelemente auf, um den Gesamt-Schmelzestrom des zweiten Polymermaterials auf die Streifen zu reduzieren.

Am Ende der Cast-Düse ist dann der Düsenaustrittsquerschnitt an dem zumindest einen zweiten Funktionsabschnitt reduziert. Wie zuvor beschrieben, kann eine solche Reduzierung durch eine Kanalquerschnittsreduzierung bis zum Düsenspalt und/oder die verstellbare Düsenlippe erreicht werden. Die Verstellung der Düsenlippe kann durch Stellschrauben oder andere Stellelemente ermöglicht werden, wobei grundsätzlich auch ein Antrieb, eine Steuerung und/oder eine Regelung solcher Stelleelemente in Betracht kommt. Beispielsweise können auch Eigenschaften der Coextrusionsverbundfolie wie die Lage der Funktionsbereiche, Dicken oder dergleichen erfasst und für eine Kontrolle und/oder Regelung genutzt werden.

Wie zuvor beschrieben, ist es möglich, die Streifen direkt in einem Kleiderbügelverteiler zu erzeugen. Grundsätzlich es aber auch möglich, Streifen des zweiten Polymermaterials bereits zuvor in einem Coextrusionsadapter zu bilden, wobei dann eine Schmelze des vollflächigen ersten Polymermaterials und des lediglich in Form von Streifen vorgesehenen zweiten Polymermaterials gemeinsam, beispielsweise in einem Kleiderbügelverteiler einer Cast-Düse, aufgeweitet werden. Die zuvor beschriebenen Maßnahmen zur Konturierung der Coextrusionsverbundfolie an Düsenspalt sind dann ebenfalls vorgesehen.

Im Rahmen der Erfindung wird die Coextrusionsverbundfolie mit zumindest einem vorzugsweise elastischen ersten Funktionsabschnitt und zumindest einem vorzugsweise nicht-elastischen zweiten Funktionsabschnitt beansprucht. Sofern gemäß einer bevorzugten Ausstattung der Erfindung mehrere erste und/oder mehrere zweite Funktionsabschnitte vorgesehen sind, so sind diese jeweils bevorzugt wie zuvor beschrieben ausgeführt. Insbesondere kann vorgesehen sein, dass die ersten und/oder die zweiten Funktionsabschnitte jeweils untereinander gleich oder im Wesentlichen gleich ausgestaltet oder zumindest ausgelegt sind. Übliche Produktionsschwankungen sind dabei nicht zu berücksichtigen.

Unter Berücksichtigung konkreter Anforderungen ist es jedoch auch möglich, bestimmte vorzugsweise elastische erste Funktionsabschnitt und/oder vorzugsweise nicht-elastischen zweite Funktionsabschnitte unterschiedlich auszugestalten.

Hinsichtlich einer bevorzugten Verwendung der Coextrusionsverbundfolie für Einweg-Hygieneartikel kann die Coextrusionsverbundfolie mit einer großen Breite von beispielsweise mehr als einem Meter gefertigt werden, wobei dann diese Coextrusionsverbundfolie mehrnutzig geschnitten wird. Insbesondere können dann aus einer solchen breiten Coextrusionsverbundfolie mit einer Vielzahl von vorzugsweise elastischen ersten Funktionsabschnitten und vorzugsweise nicht-elastischen zweiten Funktionsabschnitten einzelne streifenförmige Bahnen für die weitere Verarbeitung geschnitten werden.

In der nachfolgenden Tabelle 1 sind Ausführungsbeispiele 1 bis 5 dargestellt.

**Tabelle 1**

| | **zweites Polymermaterial** | **erstes Polymermaterial** | **maximale Dicke [µm)** | **minimale Dicke [µm]** | **Dickenverhältnis** |
|---|---|---|---|---|---|
| 1 | 75 Gew.-% SBS mit Styrol/Butadien-Verhältnis von 30:70 | 100 Gew.-% PE-LLD | 46 | 30 | 1,5 |
| | 12 Gew.-% PS | | | | |
| | 8 Gew.-% Plasticizer | | | | |
| | 5 Gew.-% PE, PE-Copolymere | MFR (200°C/5kg): 20,9 g/10min | | | |
| | MFR (200°C/5kg): 15,2 g/10min | | | | |
| 2 | 75 Gew.-% SBS mit Styrol/Butadien-Verhältnis von 30:70 | 60 Gew.-% PE-LLD | 43 | 27 | 1,6 |
| | 12 Gew.-% PS | | | | |
| | | 32 Gew.-% PP | | | |
| | 8 Gew.-% Plasticizer | | | | |
| | 5 Gew.-% PE, PE-Copolymere oder Kombinationen | 8 Gew.-% Talkum MFR (200°C/5kg): 27,0 g/10min | | | |
| | MFR (200°C/5kg): 15,2 g/10min | | | | |
| 3 | 75 Gew.-% SBS mit Styrol/Butadien-Verhältnis von 30:70 | 60 Gew.-% PE-LLD | 39 | 20 | 2,0 |
| | 12 Gew.-% PS | 32 Gew.-% PP | | | |
| | 8 Gew.-% Plasticizer | 8 Gew.-% Talkum | | | |
| | 5 Gew.-% PE, PE-Copolymere | MFR (200°C/5kg): 27,0 g/10min | | | |
| | MFR (200°C/5kg): 15,2 g/10min | | | | |
| 4 | 75 Gew.-% SBS mit Styrol/Butadien-Verhältnis von 30:70 | 80 Gew.-% PP-BC | 37 | 19 | 1,9 |
| | 12 Gew.-% PS | | | | |
| | 8 Gew.-% Plasticizer | 20 Gew.-% TPE-O | | | |
| | 5 Gew.-% PE, PE-Copolymere | MFR (200°C/5kg): 14,3 g/10min | | | |
| | MFR (200°C/5kg): 15,2 g/10min | | | | |
| 5 | 75 Gew.-% SBS mit Styrol/Butadien-Verhältnis von 30:70 | 53 Gew.-% PE-LLD | 32 | 13 | 2,5 |
| | 12 Gew.-% PS | 32 Gew.-% PP | | | |
| | | 8 Gew.-% TPE-O | | | |
| | 8 Gew.-% Plasticizer | | | | |
| | | 7 Gew.-% Talkum | | | |
| | 5 Gew.-% PE, PE-Copolymere | MFR (200°C/5kg): 23,9 g/10min | | | |
| | MFR (200°C/5kg): 15,2 g/10min | | | | |

In allen Ausführungsbeispielen ist als zweites Polymermaterial Styrol-Butadien-Styrol (SBS) mit 75 vorgesehen, während der Rest von Polystyrol (PS) üblichen Beimengungen und Füllstoffen gebildet ist. Die Eigenschaften des zweiten Polymermaterials werden dabei im Wesentlichen von dem SBS bestimmt und übliche Beimischungen und Füllstoffe sind dem Fachmann bekannt. Das Styrol/Butadien-Verhältnis beträgt 30:70, wobei die Block-Copolymere vorzugsweise symmetrisch oder im Wesentlichen symmetrisch sind. Entsprechende SBS-Typen sind beispielsweise von den Unternehmen Kraton Dynasol und TSRC erhältlich.

Bei dem ersten Ausführungsbeispiel ist das erste Polymermaterial zu 100 Gew.-% aus linearem Polyethylen niedriger Dichte (PE-LLD) mit den angegebenen Eigenschaften gebildet. Entsprechende PE-Typen sind beispielsweise von den Herstellern Dow (Dowlex), Argus (Argutex), Borealis und Exxon erhältlich.

Gemäß dem zweiten Ausführungsbeispiel ist bei dem ersten Polymermaterial das PE-LLD mit einem Anteil von 60 Gew.-% vorgesehen. Des Weiteren enthält das erste Polymermaterial wie angegeben Polypropylen (PP) und Talkum.

Das zweite und dritte Ausführungsbeispiel unterscheiden sich im Wesentlichen durch die maximale Dicke und die minimale Dicke der Coextrusionsverbundfolie.

Bei dem vierten Ausführungsbeispiel ist Polypropylen-Blockcompolymer (PP-BC) mit den aufgeführten Eigenschaften mit einem thermoplastischen Elastomer auf Olefinbasis gemischt. Das Polypropylen-Blockcompolymer ist beispielsweise von Dow (Dowlex), Argus (Argutec), Borealis oder Exxon erhältlich. Das thermoplastische Elastomer auf Olefinbasis ist beispielsweise von Exxon (Vistamaxx) oder Dow (Infuse) erhältlich.

Zu den Ausführungsbeispielen sind elastische Eigenschaften für eine Festigkeits-Zugprüfung und eine Hysterese-Messung der elastischen Eigenschaften in der Tab. 2 und 3 dargestellt. Die Messungen beziehen sich auf eine Analyse in Querrichtung, also senkrecht zu der Produktionsrichtung.

**Tabelle 2**

| Beispiel | Messposition | Festigkeit | | | | | |
|---|---|---|---|---|---|---|---|
| | | Fmax-[N] | Dehnung bei Bruch [%] | F-10% [N] | F-100% [N] | Dehnung bei 5N[%] | Dehnung bei 10N[%] |
| 1 | elastischer Abschnitt | 22,1 | 818,1 | 3,1 | 4,3 | 232,5 | 615,7 |
| 2 | elastischer Abschnitt | 31,3 | 893,0 | 4,4 | 5,6 | 20,8 | 503,9 |
| 3 | elastischer Abschnitt | 28,9 | 975,0 | 3,1 | 4,2 | 260,4 | 623,5 |
| 4 | elastischer Abschnitt | 33,6 | 946,9 | 3,9 | 5,3 | 47,0 | 512,4 |
| 5 | elastischer Abschnitt | 7,4 | 575,7 | 2,8 | 3,8 | 356,3 | - |
| 1 | nicht-elastischer Abschnitt | 23,6 | 1069,8 | 6,6 | 6,6 | 4,8 | 632,4 |
| 2 | nicht-elastischer Abschnitt | 16,5 | 724,9 | 9,5 | 9,1 | 1,7 | 156,1 |
| 3 | nicht-elastischer Abschnitt | 7,1 | 577,1 | 4,7 | 5,0 | 40,8 | - |
| 4 | nicht-elastischer Abschnitt | 16,2 | 817,9 | 6,2 | 6,1 | 4,6 | 546,7 |
| 5 | nicht-elastischer Abschnitt | 7,8 | 802,5 | 3,5 | 3,7 | 540,3 | - |

**Tabelle 3**

| Beispiel | Messposition | Hysterese | | | | | |
|---|---|---|---|---|---|---|---|
| | | Kraft-50% [N] | Kraft-100% [N] | Kraft 120% [N] | Kraft 50%U2 [N] | Kraft 75%U2 [N] | P-Set[%] |
| 1 | elastischer Abschnitt | 4,2 | 4,3 | 4,3 | 0,6 | 1,3 | 14,6 |
| 2 | elastischer Abschnitt | 5,4 | 5,6 | 5,6 | 0,6 | 1,1 | 15,3 |
| 3 | elastischer Abschnitt | 3,9 | 4,1 | 4,1 | 0,5 | 1,0 | 16,5 |
| 4 | elastischer Abschnitt | 5,0 | 5,3 | 5,3 | 0,4 | 0,9 | 17,7 |
| 5 | elastischer Abschnitt | 3,6 | 3,8 | 3,7 | 0,4 | 0,9 | 17,8 |

Der Festigkeits-Zugversuch erfolgt in Anlehnung an DIN ISO 527 (1 bis 3). Im Zugversuch werden die Werkstoffeigenschaften bei einachsiger Zugbeanspruchung und anfangs gleichmäßiger Spannungsverteilung über den Querschnitt ermittelt. Entsprechende Prüfgeräte sind von dem Unternehmen Zwick erhältlich. Die Prüfung erfolgt bei Normalklima, wobei Proben mit einer Breite von 25,4 mm bereitgestellt werden. Die Einspannlänge der Proben beträgt 30 mm. Die Prüfung erfolgt ausgehend von einer Vorkraft von 0,1 Newton durch die Vorkraft wird sichergestellt, dass das Muster ordnungsgemäß eingespannt und weitgehend kräftefrei aufgespannt ist. Die Geschwindigkeit bis zu der eingestellten Vorkraft beträgt 30 mm/min. Die Prüfung wird dann mit einer Messegeschwindigkeit von 50 mm/min bis zu einer Dehnung von 100 % und 500 mm/min ab 100 % Dehnung durchgeführt.

Die Hysterese-Messung erfolgt mit einem Testgerät Zwick Z010 bei einer Temperatur von 23 °C ±2 °C und einer Luftfeuchte von 50 % ±5 %. Die Probenbreite und die Einspannlänge betragen jeweils von 25,4 mm, wobei eine Vorkraft für den Beginn der Messung auf 0,05 Newton eingestellt ist. Die Geschwindigkeit bis zur eingestellten Vorkraft beträgt 50 mm/min. Die Messgeschwindigkeit ist für die Messung dann auf 500 mm/min eingestellt. Bei der Messung werden zwei Zyklen durchfahren, wobei der untere Umkehrpunkt auf 121 % Dehnung bezogen auf die Ausgangslänge eingestellt ist. Die Haltezeit im gedehnten Zustand beträgt 30 Sekunden. Die Haltezeit im entspannten Zustand zwischen den beiden Zyklen beträgt 60 Sekunden. Die in der Tabelle 2 und 3 angegebenen Werte werden von dem Testgerät generiert und ausgegeben. Die Werte in der Tabelle mit dem Zusatz U2 beziehen sich auf die elastische Rückstellung nach der zweiten Dehnung ("unload 2").

Die Erfindung wird nachfolgend anhand von exemplarischen Figuren erläutert. Es zeigen:
- Fig. 1: eine Coextrusionsverbundfolie,
- Fig. 2: einen Coextrusionsadapter für die Herstellung der Coextrusionsverbundfolie,
- Fig. 3a: eine Teilansicht einer Extrusionsdüse für die Herstellung der Coextrusionsverbundfolie,
- Fig. 3b: eine weitere Teilansicht der Extrusionsdüse für die Herstellung der Coextrusionsverbundfolie
- Fig. 4: eine Draufsicht auf eine Coextrusionsverbundfolie mit einem Schnittmuster zur Bildung von Decklagen für einen Einweg-Hygieneartikel,
- Fig. 5: eine aus der Coextrusionsverbundfolie gebildete Decklage,
- Fig. 6: eine alternative Ausgestaltung eine Coextrusionsverbundfolie und ein Vorprodukt für einen Einweg-Hygieneartikel mit der Coextrusionsverbundfolie,
- Fig. 7: eine weitere alternative Ausgestaltung einer Coextrusionsverbundfolie,
- Fig. 8: einen Einweg-Hygieneartikel,
- Fig. 9: ein Kraft-Dehnungsdiagram.

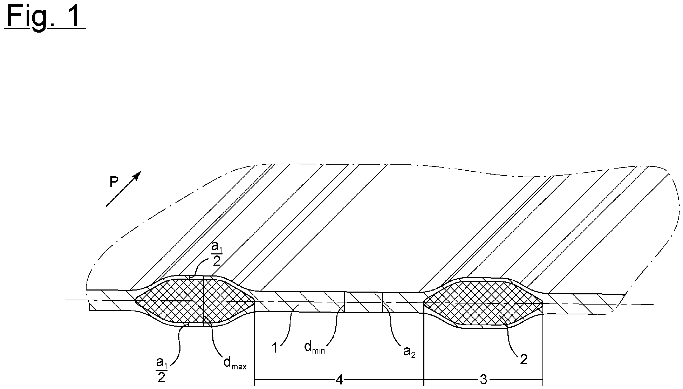

Die Fig. 1 zeigt eine Coextrusionsverbundfolie mit einem ersten Polymermaterial 1 und einem zweiten, elastischen Polymermaterial 2, wobei sich das erste Polymermaterial 1 vollflächig erstreckt und wobei das zweite, elastische Polymermaterial 2 lediglich abschnittsweise vorgesehen ist. Das zweite, elastische Polymermaterial 2 erstreckt sich in Form von Streifen entlang einer Produktionsrichtung P, wobei elastische erste Funktionsabschnitte 3 mit dem zweiten Polymermaterial 2 und in dem dargestellten Ausführungsbeispiel vorzugsweise nicht-elastische zweite Funktionsabschnitte 4 ohne das zweite Polymermaterial 2 gebildet sind.

Das Verhältnis einer an den ersten Funktionsabschnitten 3 bestimmten maximalen Foliendicke dₘₐₓ zu einer an den zweiten Funktionsabschnitten 4 bestimmten minimalen Foliendicke dₘᵢₙ beträgt zumindest 1,4:1. Das Verhältnis einer an den zweiten Funktionsabschnitten 4 bestimmten maximalen Gesamtdicke a₂ des ersten Polymermaterials 1 zu einer an den ersten Funktionsabschnitten 3 bestimmten minimalen Gesamtdicke a₁ des erstem Polymermaterials 1 beträgt zumindest 2:1.

Dabei ist zu erkennen, dass bezüglich einer Mittelebene der Coextrusionsverbundfolie eine Symmetrie vorliegt, wobei sich an den ersten Funktionsabschnitten 3 ein symmetrischer dreischichtiger Aufbau ergibt. Dort ist das zweite, elastische Polymer 2 jeweils von einer Schicht mit einer Dicke von a₁/2 des ersten Polymermaterials 1 abgedeckt.

Die entlang der Produktionsrichtung P verlaufenden Streifen, d. h. entsprechend die ersten Funktionsabschnitte 3 weisen senkrecht zu der Produktionsrichtung P eine Breite zwischen 1 cm und 12 cm auf. Der Abstand zwischen zwei ersten Funktionsabschnitten 3 und damit die Breite der zweiten Funktionsabschnitte 4 beträgt zwischen 3 cm und 24 cm.

Während sich an den ersten Funktionsabschnitten 3 ein dreischichtiger Aufbau ergibt, sind in dem dargestellten Ausführungsbeispiel die zweiten Funktionsabschnitte 4 ausschließlich von dem ersten Polymermaterial 1 gebildet.

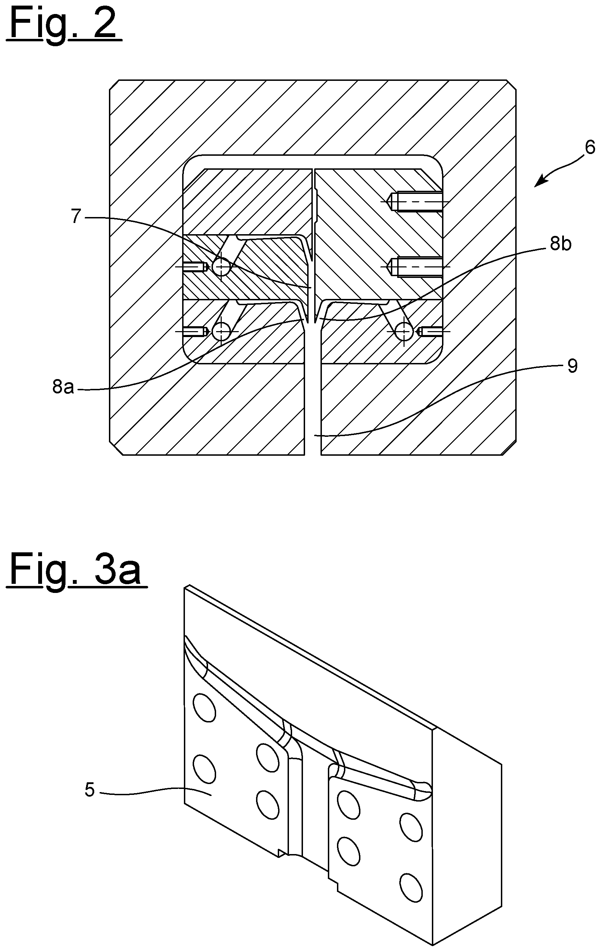

Die Coextrusionsverbundfolie kann dadurch hergestellt werden, dass einer exemplarisch in einer Teilansicht in der Fig. 3a dargestellten Extrusionsdüse 5 beispielsweise in Form einer Cast-Düse mit einem in der Fig. 2 dargestellten Coextrusionsadapter 6 (Feedblock) eine Schmelze des ersten Polymermaterial 1 vollflächig und eine Schmelze des zweiten Polymermaterials 2 lediglich bereichsweise in Form von Streifen zugeführt werden. Gemäß der Fig. 2 wird das zweite Polymermaterial 2 in Form von Streifen in einem mittleren Zuführspalt 7 zugeführt, während das erste Polymermaterial 1 durch außenliegende Zuführspalte 8a, 8b in einen Schmelzekanal 9 innerhalb des Coextrusionsadapters 6 eingebracht wird.

Das erste Polymermaterial 1 wird über die außenliegenden Zuführtspalte 8a, 8b eingebracht.

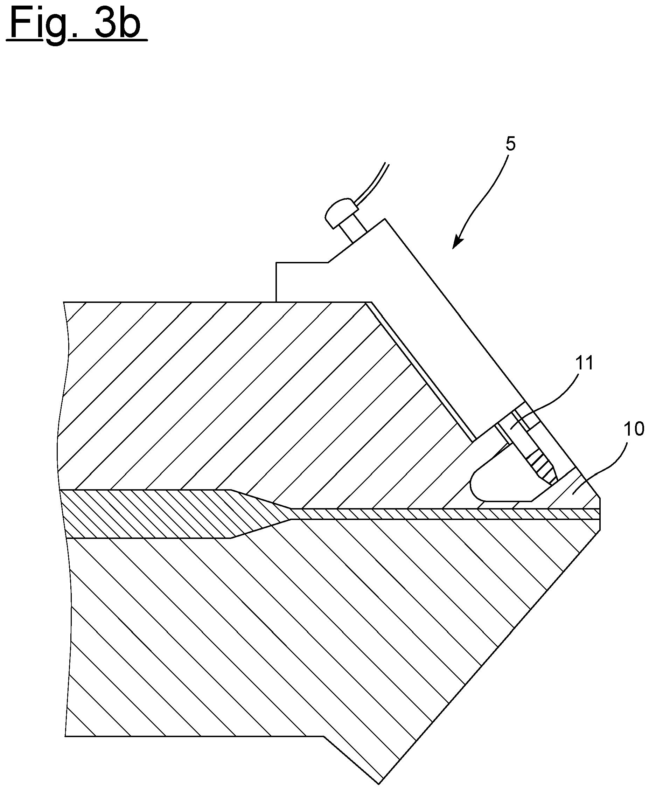

In der Extrusionsdüse 5 wird der Schmelzestrang zu der Coextrusionsverbundfolie aufgeweitet. Um das in der Fig. 1 dargestellte Dickenprofil zu erreichen, kann gemäß der Fig. 3b eine Düsenlippe 10 an der Extrusionsdüse 5 vorgesehen sein, welche durch eine Vielzahl von Einstellmitteln 11 verstellbar ist.

In dem exemplarisch dargestellten Ausführungsbeispiel werden die Streifen bereits in dem Coextrusionsadapter 6 gebildet. Alternativ kann beispielsweise auch eine Extrusionsdüse 5 mit drei übereinander angeordnete Kleiderbügelverteiler vorgesehen sein, wobei das zweite Polymermaterial 2 dem mittleren Kleiderbügelverteiler zugeführt wird. Der mittlere Kleiderbügelverteiler weist dann Stauelemente auf, um den Gesamt-Schmelzestrom des zweiten Polymermaterials 2 auf die Streifen zu reduzieren.

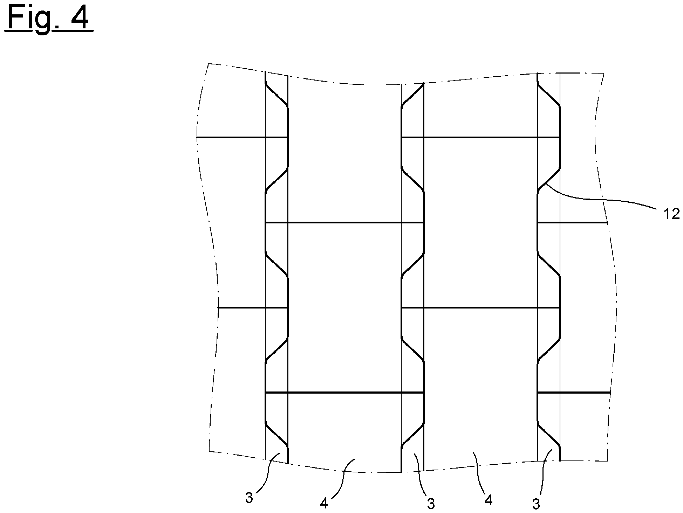

Die Fig. 4 zeigt eine Draufsicht auf die Coextrusionsverbundfolie mit einer Vielzahl von elastischen Abschnitten 3 und nicht-elastischen Abschnitten 4. Des Weiteren sind Schnittlinien 12 eingezeichnet, um aus der Coextrusionsverbundfolie Decklagen für einen Einweg-Hygieneartikel, insbesondere eine Windel zu bilden.

Die Schnittlinien 10 sind so angeordnet, dass jede Decklage 2 randseitige elastische Abschnitte 3 aufweist, die jeweils in einem mittleren Bereich durch seitliche Beineinschnitte entfernt sind.

Die elastischen Abschnitte 3 bilden dabei in den an den mittleren Bereich in Längsrichtung anschließenden Taillenbereichen der Decklage integrierte elastische Ohren. Zur Bildung einer Höschenwindel können die Abschnitte miteinander verbunden, beispielsweise verschweißt oder verklebt werden. Um eine Windel zu bilden, welche mit Verschlussmitteln angelegt werden kann, können an die integrierten elastischen Ohren Verschlussmittel, beispielsweise Kletthaken, angebracht werden.

Zur Bildung der gesamten Decklage kann die Coextrusionsverbundfolie zumindest bereichsweise mit zunächst einem Nonwoven laminiert sein.

Die Anordnung von Nonwoven kann dabei vollflächig erfolgen, sodass dann die in der Fig. 4 dargestellte Decklage auch vollflächig an einer Seite oder auch an beiden Seiten zumindest eine Schicht aus Nonwoven aufweist. Grundsätzlich ist es aber auch möglich, an einer Seite oder an beiden Seiten Nonwoven lediglich teilweise anzuordnen, um bestimmte Funktionsbereiche abzudecken, insbesondere Funktionsbereiche, welche für die Haptik, das Erscheinungsbild und/oder die Funktion der Decklage von besonderer Bedeutung sind.

Die Coextrusionsverbundfolie kann zumindest bereichsweise vorgedehnt sein. Im Bereich der nicht-elastischen Abschnitte kann eine Vordehnung beispielsweise erfolgen, um eine Atmungsaktivität zu erreichen. Dies ist beispielsweise möglich, wenn das erste Polymermaterial mit Füllstoffen versehen ist, welche bei einer Dehnung Poren bilden. Insbesondere ist es damit möglich die Decklage als Außenschicht einer Windel gleichzeitig wasserdicht und Atmungsaktivität auszubilden.

Eine auch als Aktivierung bezeichnete Vordehnung an den ersten Funktionsabschnitten 3 kann zweckmäßig sein, um danach bei der Benutzung der Windel eine leichte Dehnbarkeit zu erreichen.

Bei einem Einweg-Hygieneartikel, insbesondere in Form einer Windel, kann die Decklage somit als wasserdichte Außenlage vorgesehen sein und mit einer wasserdurchlässigen Innenlage kombiniert werden. Dabei ist üblicherweise ein absorbierender Kern zwischen der wasserdurchlässigen Innenlage und der Außenlage angeordnet.

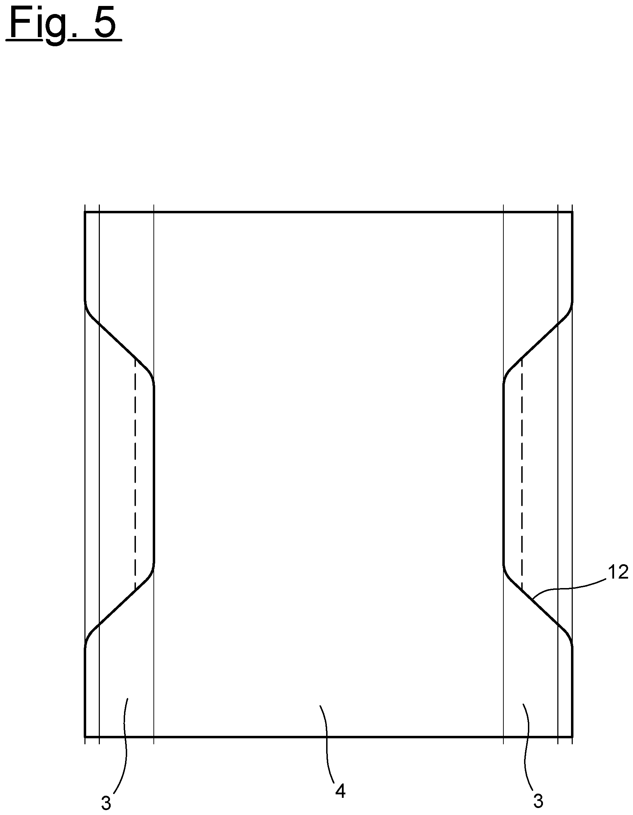

Die Fig. 5 zeigt in einer weiteren Ausführungsform eine Decklage, welche aus einer Coextrusionsverbundfolie geschnitten ist. Dabei ist auch angedeutet, dass die Schnittlinien 12 so verlaufen können, dass an den seitlichen Beineinschnitten noch ein Streifen des ersten Funktionsabschnittes 3 verbleibt, wobei auch dort dann - gegebenenfalls nach einer Aktivierung - gewisse elastische Rückstelleigenschaften erreicht werden können, um einen verbesserten Sitz an den Beinen eines Trägers zu erreichen.

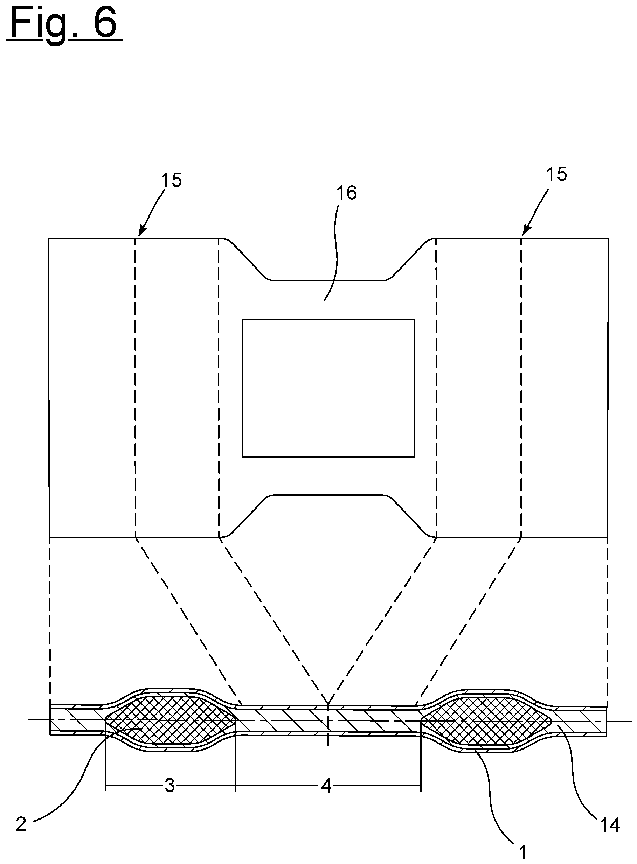

Die Fig. 6 zeigt zum besseren Verständnis in einer Abbildung einerseits eine alternative Ausgestaltung der Coextrusionsverbundfolie und ein damit gebildetes Vorprodukt für einen Einweg-Hygieneartikel 13 mit der Coextrusionsverbundfolie. Die Coextrusionsverbundfolie weist ausgehend von der Ausgestaltung nach Fig. 1 ein drittes Polymermaterial 14 auf, welches sich wie das erste Polymermaterial 1 vollflächig erstreckt. An den ersten Funktionsabschnitten 3 ergibt sich ein Aufbau mit fünf Schichten. Durch das zweite Polymermaterial 2 weisen die ersten Funktionsabschnitte 3 eine hohe Elastizität auf. Das dritte Polymermaterial 14 weist ebenfalls noch gewisse elastische Eigenschaften jedoch ein schlechteres Rückstellvermögen als das zweite Polymermaterial auf. Die zweiten Funktionsabschnitte 4 können dabei je nach Ausgestaltung noch im Sinne der Erfindung elastisch sein. Zur Unterscheidung von den ersten Funktionsabschnitten 3 können die zweiten Funktionsabschnitte 4 auch als semi-elastisch bezeichnet werden.

Die Coextrusionsverbundfolie gemäß Fig. 6 kann dazu genutzt werden, an dem Einweg-Hygieneartikel 13 in Form einer Windel Bereich unterschiedlicher Elastizität bereitzustellen. Die Coextrusionsverbundfolie kann dazu wie dargestellt zugeschnitten und angeordnet werden, dass die bei der Coextrusionsverbundfolie in der Produktionsrichtung verlaufenden ersten und zweiten Funktionsabschnitte 3, 4 an dem Vorprodukt entlang von Querrändern verlaufen. Mit der dargestellten und in seiner Mitte zerschnittenen Ausschnitt der Coextrusionsverbundfolie kann ein vorderer und ein hinterer Bündchenbereich 15 des Einweg-Hygieneartikel 13 gebildet werden, wobei ein mittlerer Abschnitt 16 mit einem saugfähigen Kern von einem separaten Material gebildet ist.

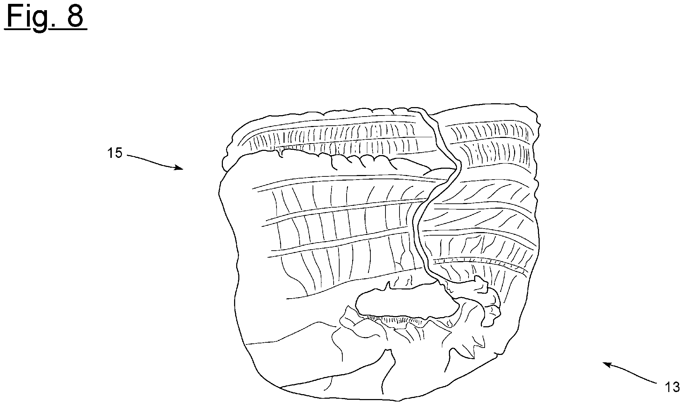

Der Einweg-Hygieneartikel 13 ist in der Fig. 8 dargestellt, wobei der vordere und der hintere Bündchenbereich 15 miteinander verbunden sind.

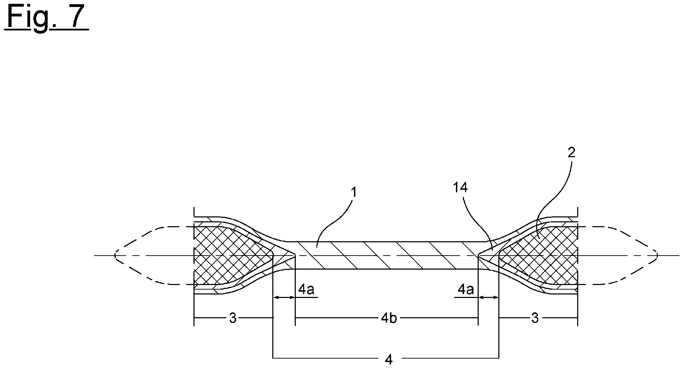

Die Fig. 7 zeigt eine weitere Ausgestaltung der der Coextrusionsverbundfolie mit dem ersten, zweiten und dritten Polymermaterial 1, 2, 14. Das dritte Polymermaterial 14 ist in Form von Streifen ausgeführt, welche aber breiter als die Streifen des zweiten Polymermaterials sind. Damit ist es beispielsweise möglich, die den zweiten Funktionsabschnitt 4 in einen noch elastische bzw. wie zuvor beschrieben semi-elastischen Teilbereich 4a und einen nicht-elastischen Teilbereich 4b aufzuteilen. Der in der Fig. 8 dargestellt Einweg-Hygieneartikel 13 kann dann einen durchgehenden Zuschnitt der Coextrusionsverbundfolie als Außenschicht aufweisen, wobei dann der nicht-elastische Teilbereich 4b den saugfähigen Kern (vgl. Fig. 6) aufnimmt.

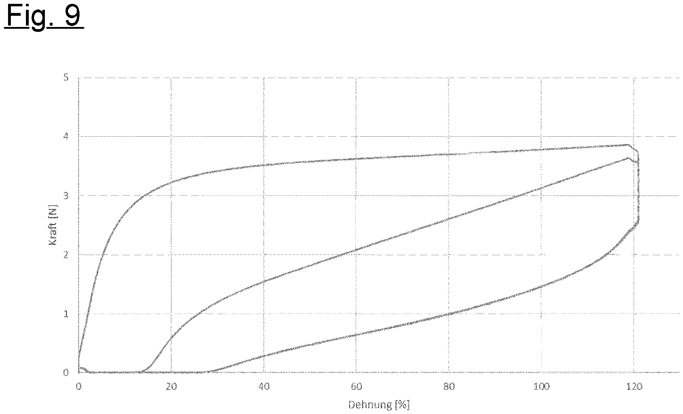

Die Fig. 9 zeigt exemplarisch ein ein Kraft-Dehnungsdiagram für den ersten elastischen Funktionsabschnitt der fünften Ausführungsbeispiels gemäß der Tabelle 1 bei der beschriebenen Hysterese-Messung. Die oberste Kurve zeigt das Verhalten bei der ersten Dehnung. Die mittlere Kurve zeigt das Verhalten bei der zweiten Dehnung. Für die Rückstellung des Materials ergibt sich für beide Zyklen ein im Wesentlichen gleiches Verhalten, so dass die entsprechenden unteren Kurven übereinander liegen.

## Patentansprüche

1. Decklage für einen Einweg-Hygieneartikel, insbesondere eine Windel, umfassend eine Coextrusionsverbundfolie mit einem ersten vollflächigen Polymermaterial (1) und einem zweiten, elastischen Polymermaterial (2) , welches lediglich bereichsweise in Form zumindest eines Streifens zur Ausbildung zumindest eines elastischen ersten Funktionsabschnittes (3) mit dem zweiten, elastischen Polymermaterial (2) und zumindest eines zweiten Funktionsabschnitts (4) ohne das zweite, elastische Polymermaterial (2) angeordnet ist, **dadurch gekennzeichnet, dass** die Coextrusionsverbundfolie an dem zumindest einen Streifen gegenüber dem zumindest eines zweiten Funktionsabschnitts (4) eine erhöhte Dicke aufweist.

2. Decklage nach Anspruch 1, **dadurch gekennzeichnet, dass** der zumindest eine zweite Funktionsabschnitt (4) nicht-elastisch ist.

3. Decklage nach Anspruch 1 oder 2, **dadurch gekennzeichnet, dass** das Verhältnis einer an dem ersten Funktionsabschnitt (3) bestimmten maximalen Foliendicke (dₘₐₓ) zu einer an dem zweiten Funktionsabschnitt (4) bestimmten minimalen Foliendicke (dₘᵢₙ) zumindest 1,4:1 beträgt und/oder dass das Verhältnis einer an dem zweiten Funktionsabschnitt (4) bestimmten maximalen Gesamtdicke (a₂) des ersten Polymermaterials (1) zu einer an dem ersten Funktionsabschnitt (3) bestimmten minimalen Gesamtdicke (a₁) des ersten Polymermaterials (1) zumindest 2:1 beträgt.

4. Decklage nach einem der Ansprüche 1 bis 3, **dadurch gekennzeichnet, dass** das zweite, elastische Polymermaterial (2) in Form von genau zwei zu in einer Längsrichtung verlaufenden Längsrändern benachbarten Streifen angeordnet ist, wobei in einem entlang der Längsrichtung mittleren Bereich durch seitliche Beineinschnitte die Streifen zumindest teilweise entfernt sind.

5. Decklage nach Anspruch 4, **dadurch gekennzeichnet, dass** die Streifen in zumindest einem an dem mittleren Bereich in Längsrichtung anschließenden Taillenbereich in die Decklage integrierte elastische Ohren bilden.

6. Decklage nach einem der Ansprüche 1 bis 5, **dadurch gekennzeichnet, dass** die Coextrusionsverbundfolie zumindest bereichsweise mit einem Nonwoven laminiert ist.

7. Decklage nach einem de Ansprüche 1 bis 6, **dadurch gekennzeichnet, dass** die Coextrusionsverbundfolie zumindest bereichsweise vorgedehnt ist.

8. Coextrusionsverbundfolie nach einem der Ansprüche 1 bis 7, **dadurch gekennzeichnet, dass** der zumindest eine Streifen ein Breite zwischen 1 cm und 12 cm aufweist.

9. Coextrusionsverbundfolie nach einem der Ansprüche 1 bis 8, **dadurch gekennzeichnet, dass** zumindest zwei elastische erste Funktionsabschnitte (3) in Form von Streifen vorgesehen sind, wobei der Abstand zwischen den Streifen zwischen 3 cm und 24 cm beträgt.

10. Einweg-Hygieneartikel, insbesondere Windel, mit einer Decklage nach einem der Ansprüche 1 bis 9.

11. Einweg-Hygieneartikel nach Anspruch 10 mit der Decklage als wasserdichte Außenlange oder Teilbereich einer wasserdichten Außenlage, mit einer wasserdurchlässigen Innenlage und mit einem zwischen der Außenlage und der Innenlage angeordneten absorbierenden Kern.

12. Einweg-Hygieneartikel nach Anspruch 10 oder 11 in Form einer Windel, wobei die Decklage an dem zumindest einen elastischen Abschnitt der Coextrusionsverbundfolie in einem Taillenbereich ein integriertes elastisches Bünden oder Windelohr bildet.

13. Einweg-Hygieneartikel nach Anspruch 12 mit zwei Windelohren, an denen jeweils ein Verschlussmittel, insbesondere mit Kletthaken, angeordnet ist.

14. Einweg-Hygieneartikel nach einem der Ansprüche 10 bis 13, wobei der zumindest eine Streifen entlang einer Längsrichtung eines Windelkörpers von einem vorderen Bündchenbereich über einen mittleren Bereich zu einem hinteren Bündchenbereich verläuft.

15. Einweg-Hygieneartikel nach Anspruch 14, wobei zwei Streifen benachbart zu Längsrändern entlang der Längsrichtung verlaufen und wobei die Streifen im mittleren Bereich zumindest teilweise entfernt sind.

16. Einweg-Hygieneartikel nach einem der Ansprüche 10 bis 13, wobei der zumindest eine Streifen entlang einer Querrichtung eines Windelkörpers an einem vorderen Bündchenbereich und/oder an einem hinteren Bündchenbereich verläuft.
